(19) Europäisches Patentamt
European Patent Office
Office européen des brevets

(11) Veröffentlichungsnummer: **0 078 234**
**B1**

# (12) EUROPÄISCHE PATENTSCHRIFT

(45) Veröffentlichungstag der Patentschrift: **04.07.90**

(51) Int. Cl.⁵: **C 07 D 213/61,** C 07 C 47/14

(21) Anmeldenummer: **82810429.9**

(22) Anmeldetag: **14.10.82**

## (54) 5-Halogenalkyl-pyridine.

(30) Priorität: **20.10.81 CH 6692/81**
**29.12.81 CH 8328/81**
**01.09.82 CH 5195/82**

(43) Veröffentlichungstag der Anmeldung:
**04.05.83 Patentblatt 83/18**

(45) Bekanntmachung des Hinweises auf die Patenterteilung:
**04.07.90 Patentblatt 90/27**

(84) Benannte Vertragsstaaten:
**AT BE CH DE FR GB IT LI NL**

(56) Entgegenhaltungen:
**EP-A-0 004 414**
**DE-A-3 008 081**
**GB-A-2 002 368**
**US-A-3 329 707**

**Houben-Weyl "Methoden der organischen Chemie" 5/1b (1972), Seite 451**

**Houben-Weyl "Methoden der organischen Chemie", 5/4 (1972), 729, 731 und 734**

(73) Patentinhaber: **CIBA-GEIGY AG**
**Klybeckstrasse 141**
**CH-4002 Basel (CH)**

(72) Erfinder: **Martin, Pierre, Dr.**
**Meisenweg 38**
**CH-4310 Rheinfelden (CH)**
Erfinder: **Steiner, Eginhard, Dr.**
**Obere Hofackerstrasse 3**
**CH-4414 Füllinsdorf (CH)**

**EP 0 078 234 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neuartige substituierte 5-Halogenalkyl-pyridine und Verfahren zu deren Herstellung sowie die dabei intermediär hergestellten oder entstehenden neuen Zwischenprodukte.

Die neuen 5-Halogenalkyl-pyridine entsprechen der Formel I

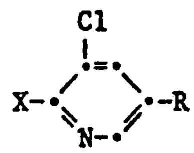

(I),

worin R einen einheitlich oder uneinheitlich mit 1 bis 21 Halogenatomen substituierten $C_2$—$C_{10}$-Alkylrest und X Halogen bedeuten. Unter Halogenatomen bzw. Halogen sind dabei vorzugsweise Fluor, Chlor oder Brom zu verstehen.

Hervorzuheben sind diejenigen Verbindungen der Formel I, worin R einen einheitlich oder uneinheitlich mit 1 bis 5 Fluor- oder Chloratomen substituierten Aethylrest und X Fluor oder Chlor bedeuten, insbesondere diejenigen, worin R einen der Reste $—CH_2—CF_3$, $—CF_2—CF_2Cl$, $—CF_2—CFCl_2$, $—CCl_2—CCl_3$, $—CF_2—CCl_3$, $—CF_2—CH_3$, $—CCl_2—CH_3$, $—CF_2—CF_3$, $—CH_2—CH_2Cl$, $—CH_2—CHCl_2$ oder $—CH_2—CCl_3$ bedeutet.

Besonders bevorzugt sind Verbindungen der Formel I, worin X Chlor bedeutet.

Die Verbindungen der Formel I können über eine oder mehrere Zwischenstufen als Ausgangsprodukte zur Herstellung von verschiedenartigen Wirkstoffen, insbesondere Wirkstoffen im Pflanzenschutz, wie z.B. Insektiziden, Herbiziden oder Fungiziden, verwendet werden. Man kann beispielsweise ausgehend von Verbindungen der Formel I zu wertvollen Insektiziden der Formel

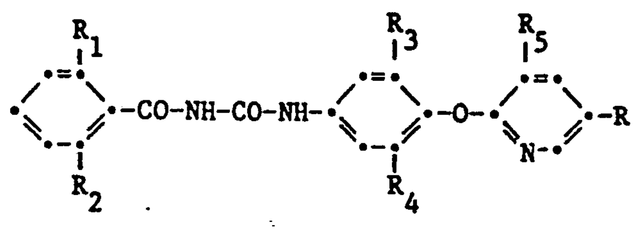

gelangen, in welcher Formel $R_1$ und $R_2$ Wasserstoff, Methyl oder Halogen, $R_3$ und $R_4$ Wasserstoff oder Halogen, $R_5$ Chlor bedeutet und R die unter Formel I vorstehend angegebene Bedeutung hat.

Durch Trichlormethyl- oder Trifluormethylgruppen substituierte Chlorpyridine konnten bisher nur durch aufwendige mehrstufige Verfahren hergestellt werden. So entstehen bei der Chlorierung des 3-Methylpyridins im allgemeinen neben der gewünschten Verbindung mehrere Isomere. Durch Chlorierung des 2,3-Dichlor-5-methylpyridins erhält man das 2,3-Dichlor-5-trichlormethylpyridin, das durch Austausch der Chloratome der Trichlormethylgruppe gegen Fluoratome in das 2,3-Dichlor-5-trifluormethyl-pyridin übergeführt werden kann (vgl. z.B. die europäische Patentveröffentlichung 004414). In ähnlicher Weise werden gemäss der deutschen Offenlegungsschrift 2 812 607 2-Halogen-5-trifluormethylpyridine und das 2,3-Dichlor-5-trifluormethylpyridin hergestellt. Halogensubstituierte 5-Trifluormethyl- und 5-difluormethylpyridine und deren Herstellung sind ferner aus der veröffentlichten britischen Patentanmeldung 2 002 368 bekannt.

Die Verbindungen der Formel I werden erfindungsgemäss durch Umsetzung eines Aldehyds der Formel II mit Acrylnitril hergestellt, wobei sich zunächst durch Anlagerungsreaktion ein Zwischenprodukt

der Formel III bildet, welches dann — gegebenenfalls in situ — zu einer Pyridin-Verbindung der Formel I cyclisiert wird:

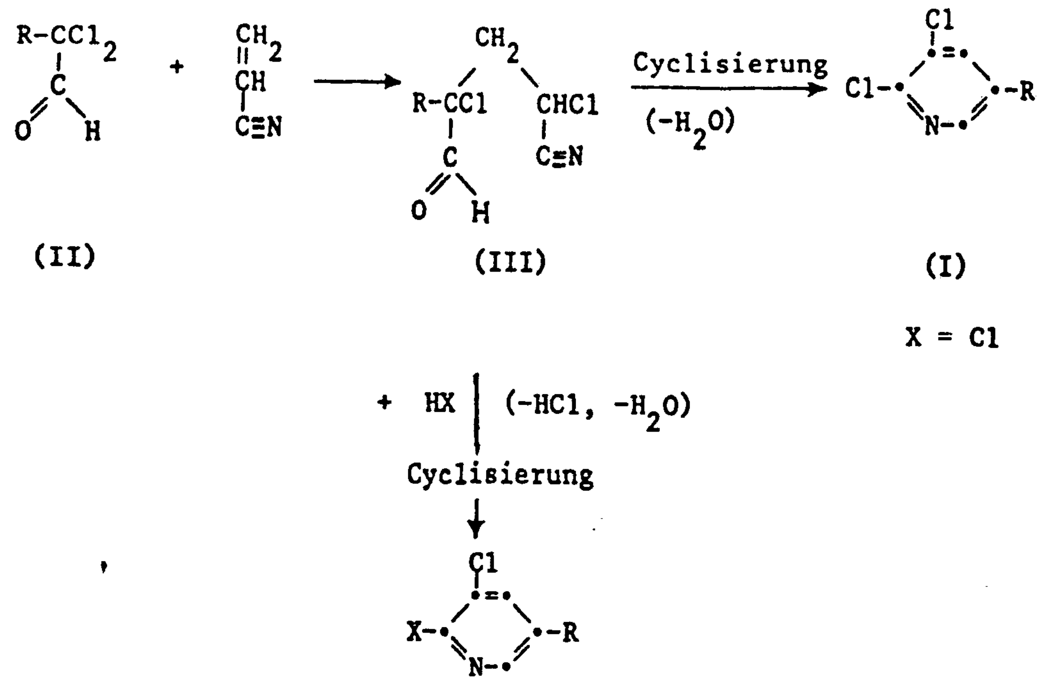


(II) (III) (I)

(I)

Wie aus dem obigen Reaktionsschema ersichtlich, gelangt man, wenn man in situ, d.h. ohne Isolierung des Zwischenproduktes der Formel III, worin X für Chlor steht, arbeitet, zu Verbindungen der Formel I, worin X Chlor bedeutet. Falls ein Zwischenprodukt der Formel III isoliert wird, besteht die Möglichkeit, dasselbe mit einem Halogenwasserstoff HX umzusetzen, um zu einer Verbindung der Formel I zu gelangen, worin X nicht nur Chlor, sondern auch Fluor oder Brom bedeuten kann. Man kann ferner eine erhaltene Verbindung der Formel I im Rest R gegebenenfalls weiter halogenieren, vorzugsweise chlorieren, oder im Rest R befindliche Halogenatome durch andere Halogenatome, vorzugsweise Chlor- gegen Fluoratome, austauschen, um zu entsprechenden weiteren Verbindungen der Formel I zu gelangen. In den obigen Formeln II und III hat R die unter Formel I angegebene Bedeutung.

Als Ausgangsprodukte bevorzugt werden Aldehyde der Formel II, worin R einer der Reste $—CH_2—CCl_3$, $—CH_2—CHCl_2$ oder $—CH_2—CH_2Cl$ bedeutet. Die aus diesen bevorzugten Aldehyden durch Addition von Acrylnitril und Cyclisierung des Additionsproduktes der Formel III erhaltenen Pyridine der Formel I lassen sich in besonders vorteilhafter Weise zum 2,3-Dichlor-5-(pentachloräthyl)-pyridin chlorieren, worauf bevorzugt im Falle der letztgenannten Verbindung in der Pentachloräthyl-Seitenkette befindliche Chloratome mit Hilfe üblicher Fluorierungsmethoden durch Fluoratome ausgetauscht werden können. Die Anzahl der durch Austausch eingeführten Fluoratome hängt von den gewählten Fluorierungsbedingungen ab.

Eine formal ähnliche Reaktionsfolge von Trichlorformylbutylonitril zu 2,3,5-Trichlorpyridin ist in der europäischen Patentveröffentlichung Nr. 12117 beschrieben. Die erfindungsgemässe Cyclisierung unter Aromatisierung zu den Pyridinen der Formel I war jedoch nicht vorauszusehen. Vielmehr wäre die Bildung eines 2-Pyridon-Derivates zu erwarten gewesen. Der erfindungsgemässe Reaktionsverlauf ist daher als in hohem Masse überraschend zu bezeichnen.

Die Aldehyde der Formel II und die Verfahren zu deren Herstellung bilden, insofern sie neu sind, ebenfalls einen Gegenstand der vorliegenden Erfindung. Neue Aldehyde der Formel II können erhalten werden durch Umsetzung von Trichloracetaldehyd mit entsprechenden äthylenisch ungesättigten Verbindungen, wie z.B.:

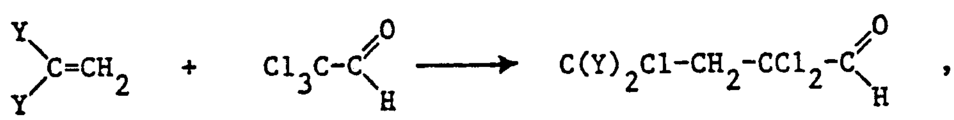

wobei Y Wasserstoff oder Halogen, vorzugsweise Chlor, bedeutet. Arbeitsweisen zur Herstellung vorwiegend längerkettiger Halogencarbonylverbindungen sind aus der US-Patentschrift 3.329.707

bekannt, wobei unter Verwendung von Eisensalzen als Katalysatoren α-Dihalogencarbonylverbindungen, wie z.B. α-dihalogenierte Aldehyde, Ketone, Carbonsäureester oder -halogenide, mit äthylenisch ungesättigten Verbindungen umgesetzt werden.

Die Anlagerungsreaktionen unter Bildung einer Verbindung der Formel III können in offenem oder geschlossenem System, bevorzugt bei einer Temperatur von 70—160°C, durchgeführt werden. Bevorzugt erfolgt die Anlagerung in geschlossenem System bei einem der angewendeten Reaktionstemperatur entsprechenden Druck, der beispielsweise im Bereich von 1—30 bar liegen kann.

Als Katalysatoren für die Anlagerungsreaktionen können erfindungsgemäss Metalle der Hauptgruppe VIII und der Nebengruppe VIa, VIIa, IB und IIb des periodischen Systems, z.B. Eiesen, Kobalt, Nickel, Ruthenium, Palladium, Chrom, Molybdän, Mangan, Kupfer und Zink verwendet werden. Diese Metalle können in elementarer Form oder in Form geeigneter Verbindungen eingesetzt werden, beispielsweise Oxide und Salze, wie Halogenide, Sulfate, Sulfite, Sulfide, Nitrate, Acetate, Stearate, Citrate, Carbonate, Cyanide und Rhodanide, sowie Komplexe mit Liganden, wie Phosphinen, Phosphiten, Benzoyl- und Acetylacetonaten, Nitrilen, Isonitrilen und Kohlenmonoxid.

Als Beispiele seien genannt: Kupfer(II)oxid, Eisen(III)oxid; Kupfer(I)-, Kupfer(II)-, Eisen(II)- und Eisen(III)bromide, -jodide und vor allem -chloride, Zinkchlorid, sowie die Chloride des Rutheniums, Rhodiums, Palladiums, Kobalts und Nickels; Kupfer(II)sulfat, Eisen(II)- und Eisen(III)sulfat; Kupfer(II)nitrat und Eisen(III)nitrat; Mangan(III)acetat, Kupfer(II)acetat, Kupfer(II)stearat, Eisen(III)citrat, Kupfer(I)cyanid; Ruthenium(II)dichloro-tris-triphenylphosphin, Rhodiumdichloro-tris-triphenylphosphin; Chrom- und Nickelacetylacetonat, Kupfer(II)acetylacetonat, Eisen(III)acetylacetonat, Kobalt(II)- und Kobalt(III)acetylacetonat, Mangan(II)acetylacetonat, Kupfer(II)benzoylacetonat; Eisencarbonyl-cyclopentadienylkomplex; Molybdäncarbonylcyclopentadienylkomplex, Chromtricarbonylarylkomplexe, Ruthenium(II)acetatkomplex, Chrom- und Molybdänhexacarbonyl, Nickeltetracarbonyl, Eisenpentacarbonyl, Kobalt- und Mangancarbonyl.

Es können auch Gemische der genannten Metalle mit Metallverbindungen und/oder anderen Zusätzen verwendet werden, wie Kupferpulver in Kombination mit einer der vorerwähnten Kupferverbindungen; Gemische von Kupferpulver mit Lithiumhalogeniden, wie Lithiumchlorid, oder Isocyaniden, wie tert.-Butylisocyanid; Gemische von Eisenpulver mit Eisen(III)chlorid, gegebenenfalls unter Zusatz von Kohlenmonoxid; Gemische von Eisen(III)chlorid mit Benzoin; Gemische von Eisen(II)- oder Eisen(III)chlorid mit Trialkylphosphiten; Gemische von Eisenpentacarbonyl und Jod.

Besonders bevorzugt sind Kupferpulver, Kupferbronze, Kupfer(I)-allem Eisen(II)- und Eisen(III)chlorid, sowie Eisenpulver; Ruthenium(III)chlorid, Ruthenium(II)dichloro-tris-triphenylphosphin, Kupferpulver, Kupferbronze, Kupfer(I)- und Kupfer(II)salze und komplexe, wie Kupfer(I)chlorid, Kupfer(II)chlorid, Kupfer(I)bromid, Kupfer(II)bromid; Kupfer(II)acetat, Kupfer(II)acetylaceton, Kupfer(II)benzoylacetonat, Kupfer(II)sulfat, Kupfer(II)nitrat, Kupfer(I)cyanid und Kupfer(I)jodid.

Ganz besonders bevorzugt sind Kupferpulver, Kupferbronze, Kupfer(I)- und Kupfer(II)chlorid bzw. -bromid und Kupfer(I) jodid, sowie deren Gemische.

Die Katalysatoren werden im allgemeinen in Mengen von etwa 0,01 bis 10 Mol%, bevorzugt 0,1 bis 5 Mol%, bezogen auf den Aldehyd, verwendet.

Die Anlägerung der Aldehyde der Formel II an Acrylnitril wird zweckmässig in Gegenwart eines inerten organischen Lösungsmittels vorgenommen. Geeignete Lösungsmittel sind solche, in denen die Katalysatoren ausreichend löslich sind oder die mit den Katalysatoren Komplexe bilden können, die aber gegenüber den Reaktionspartnern inert sind. Als Beispiele für geeignete Lösungsmittel seien genannt: Alkancarbonsäurenitrile, insbesondere solche mit 2—5 Kohlenstoffatomen, wie Acetonitril, Propionitril und Butyronitril; 3-Alkoxypropionitrile mit 1—2 Kohlenstoffatomen in der Alkoxygruppe, wie 3-Methoxypropionitril und 3-Aethoxypropionitril; aromatische Nitrile, vor allem Benzonitril; aliphatische Ketone mit vorzugsweise insgesamt 3—8 Kohlenstoffatomen, wie Aceton, Diäthylketon, Methylisopropylketon, Diisopropylketon, Methyl-tert.-butylketon; Alkyl- und Alkoxyalkylester von aliphatischen Monocarbonsäuren mit insgesamt 2—6 Kohlenstoffatomen, wie Ameisensäuremethyl- und -äthylester, Essigsäuremethyl-, -äthyl-, -n-butyl- und -isobutylester, sowie 1-Acetoxy-2-methoxyäthan; cyclische Aether, wie Tetrahydrofuran, Tetrahydropyran und Dioxan; Dialkyläther mit je 1—4 Kohlenstoffatomen in den Alkylgruppen, wie Diäthyläther, Di-n-propyläther und Diisopropyläther; N,N-Dialkylamide von Alkancarbonsäuren mit 1—3 Kohlenstoffatomen in der Alkylgruppe, wie N,N-Dimethylformamid, N,N-Dimethylacetamid, N,N-Diäthylacetamid und N,N-Dimethylmethoxyacetamid; Aethylenglykol- und Diäthylenglykoldialkyläther mit je 1—4 Kohlenstoffatomen in den Alkylgruppen, wie Aethylenglykoldimethyl-, -diäthyl- und -di-n-butyläther; Diäthylenglykoldiäthyl- und -di-n-butyläther; Phosphorsäure-tris-N,N-dimethylamid (Hexametapol). Ferner kann überschüssiges Acrylnitril als Lösungsmittel verwendet werden.

Bevorzugte Lösungsmittel für die Anlagerungsreaktion sind Alkancarbonsäurenitrile mit 2—5 Kohlenstoffatomen und 3-Alkoxypropionitrile mit 1—2 Kohlenstoffatomen in der Alkoxygruppe, insbesondere Acetonitril, Butyronitril und 3-Methoxypropionitril, oder das als Reaktand verwendete Acrylnitril.

Die Anlagerungsprodukte der Formel III sind neu, für die Synthese der Verbindungen der Formel I entwickelt worden und bilden ebenfalls einen Gegenstand der Erfindung.

Die Cyclisierung der Verbindungen der Formel III kann in einem offenen oder einem geschlossenen

System bei Temperaturen zwischen etwa 0 und 220°C, insbesondere zwischen etwa 100 und 200°C, durchgeführt werden. Vorzugsweise wird die Cyclisierung in einem offenen System durchgeführt. Bei der Cyclisierung in einem offenen System ist es vorteilhaft, diese in Gegenwart von Halogenwasserstoff oder in Gegenwart von Substanzen, welche unter den Reaktionsbedingungen Halogenwasserstoff bilden, wie Phosgen, Bortrichlorid, Aluminiumchlorid, Trialkylammoniumchloriden mit je 1—4 Kohlenstoffatomen in den Alkylgruppen, Phosphorpentachlorid, Phosphoroxychlorid oder Phosphortrichlorid, bzw. den entsprechende Brom- oder F-Verbindungen, vorzunehmen. Vorzugsweise wird die Cyclisierung in Gegenwart von Chlor-, Brom- oder Fluorwasserstoff vorgenommen.

Die Cyclisierung wird bevorzugt ohne Zusatz eines Lösungsmittels, in der Flüssig- oder in der Gasphase durch blosses Erhitzen der Verbindungen der Formel III durchgeführt. Sie kann aber auch in Gegenwart eines organischen Lösungsmittels vorgenommen werden. Als organische Lösungsmittel eignen sich beispielsweise chlorierte aliphatische Kohlenwasserstoffe, wie Chloroform, Methylenchlorid und Tetrachloräthan; gegebenenfalls chlorierte aromatische Kohlenwasserstoffe, wie Benzol, Toluol, Xylole und Chlorbenzole; N,N-Dialkylamide von Alkancarbonsäuren mit 1—3 Kohlenstoffatomen, wie N,N-Dimethylformamid, N,N-Dimethylacetamid, N,N-Diäthylacetamid und N,N-Dimethylmethoxyacetamid; cyclische Amide, wie N-Methyl-2-pyrrolidon, N-Acetyl-2-pyrrolidon und N-Methyl-ε-caprolactam; Amide der Kohlensäure, wie Tetramethylharnstoff und Dimorpholinocarbonyl; Amide der phosphorigen Säure, der Phosphorsäure, der Phenylphosphonsäure oder von Alkylphosphonsäuren mit 1—3 Kohlenstoffatomen in der Alkylgruppe, wie Phosphorsäuretriamid, Phosphorsäure-tris-(N,N-dimethylamid), Phosphorsäuretrimorpholid, Phosphorsäuretripyrrolinid, Phosphorigsäure-tris-(N,N-dimethylamid), Methanphosphonsäure-bis-(N,N-dimethylamid); Amide der Schwefelsäure oder von aliphatischen oder aromatischen Sulfonsäuren, wie Tetramethylsulfamid, Methansulfonsäuredimethylamid, oder p-Toluolsulfonsäureamid; aliphatische Ketone, cyclische Aether, Dialkyläther sowie Aethylenglykol- und Diäthylenglykoldialkyläther der vorerwähnten Art, sowie Phosphortrichlorid und Phosphoroxychlorid.

Bevorzugte Lösungsmittel für die Cyclisierung sind Chloroform, Methylenchlorid, cyclische Aether und Dialkyläther mit je 1—4 Kohlenstoffatomen in den Alkylgruppen, insbesondere Dioxan und Diäthyläther, sowie N,N-Dialkylamide von Alkancarbonsäuren mit 1—3 Kohlenstoffatomen, insbesondere N,N-Dimethylformamid.

Das erfindungsgemässe Verfahren kann vorteilhaft in der Weise durchgeführt werden, dass man die durch die Anlagerung gebildeten Verbindungen der Formel III zunächst isoliert und anschliessend in einer zweiten Verfahrensstufe cyclisiert. Dabei werden die einzelnen Verfahrensstufen wie vorstehend beschrieben durchgeführt.

Gemäss einer vorteilhaften Ausführungsform des erfindungsgemässen Verfahrens werden die Aldehyde der Formel II bei einer Temperatur von 70—160°C in Acetonitril, Butyronitril oder 3-Methoxypropionitril als Lösungsmittel in Gegenwart von 0,1—6 Mol% Kupferpulver, Kupferbronze-, Kupfer(I)- oder Kupfer(II)chlorid bzw. -bromid oder Kupfer(I)jodid in Gegenwart eines Gemisches dieser Substanzen in einem geschlossenen System mit Acrylnitril umgesetzt. Die nach Abtrennung des Lösungsmittels erhaltenen Verbindungen der Formel III werden bei einer Temperatur zwischen 100 und 200°C in einem offenen System in Gegenwart von Halogenwasserstoff oder einer Substanz, welcher unter den Reaktionsbedingungen Halogenwasserstoff bildet, zu Verbindungen der Formel I cyclisiert.

Man kann jedoch, wenn X Chlor bedeuten soll, vorteilhafterweise auch auf die Isolierung der Anlagerungsprodukte der Formel III verzichten und Additions- sowie Cyclisierungsreaktion in einem Arbeitsgang durchführen. In diesem Fall erfolgt die Umsetzung der Aldehyde der Formel II mit Acrylnitril zu den Pyridinen der Formel I bevorzugt bei einer Temperatur zwischen 70 und 220°C, insbesondere zwischen 130 und 200°C. Dabei kann in offenem oder geschlossenem System gearbeitet werden. Wird die Umsetzung in offenem System durchgeführt, so kann es zweckmässig sein, diese in Gegenwart von Chlorwasserstoff oder in Gegenwart von Substanzen vorzunehmen, welche unter den Reaktionsbedingungen Chlorwasserstoff bilden. Derartige Substanzen sind beispielsweise Phosgen, Bortrichlorid, Aluminiumchlorid, Trialkylammoniumchloride mit je 1—4 Kohlenstoffatomen in den Alkylgruppen, Phosphorpentachlorid, Phosphoroxychlorid oder Phosphortrichlorid. Die einstufige Herstellung von Pyridinen der Formel I erfolgt aber vorzugsweise in einem geschlossenen System bei einem der jeweiligen Reaktionstemperatur entsprechenden Druck, der beispielsweise je nach Reaktionstemperatur im Bereich von 1—50 bar liegen kann. Besonders bevorzugt ist die einstufige Synthese von Verbindungen der Formel I in einem geschlossenen System bei einem Druck von 1—30 bar.

Diese einstüfige Synthese kann ebenfalls in Gegenwart eines Katalysators und zweckmässig in Gegenwart eines inerten organischen Lösungsmittels durchgeführt werden. Als Katalysatoren und Lösungsmittel kommen solche der eingangs beschriebenen Art in Betracht, wobei in Bezug auf bevorzugte Katalysatoren und Katalysatormengen das im vorangehenden Erwähnte gilt.

Bevorzugte Lösungsmittel für die einstufige Durchführung des Verfahrens sind Alkancarbonsäurenitrile mit 2—5 Kohlenstoffatomen und 3-Alkoxypropionitrile mit 1—2 Kohlenstoffatomen in der Alkoxygruppe. Besonders geeignete Lösungsmittel sind Acetonitril, Butyronitril und 3-Methoxypropionitril oder ein Ueberschuss des als Reaktand verwendeten Acrylnitrils. Nach Beendigung der Reaktion können die Chlorpyridine der Formel I auf übliche Weise, z.B. durch Abdampfen

des Lösungsmittels und Reinigung der Rohprodukts durch Destillation oder Wasserdampfdestillation, isoliert werden.

Gemäss einer weiteren vorteilhaften Ausführungsform des erfindungsgemässen Verfahrens setzt man die Aldehyde der Formel II und das Acrylnitril, in Acetonitril, Butyronitril oder 3-Methoxypropionitril als Lösungsmittel in Gegenwart von 0,1 bis 6 Mol% Kupferpulver, Kupferbronze, Kupfer(I)- oder Kupfer(II)chlorid bzw. -bromid oder Kupfer(I)jodid oder einem Gemisch dieser Substanzen bei 130—200°C in einem geschlossenen System bei einem der jeweils angewandten Reaktionstemperatur entsprechenden Druck direkt zu den Pyridinen der Formel I um.

Die nach den vorstehend beschriebenen Verfahren hergestellten Verbindungen der Formel I können gegebenenfalls durch weitere Halogenierung der am Pyridinring befindlichen Seitenkettengruppe R oder durch Austausch der in dieser Gruppe R befindlichen Halogenatome durch andere Halogenatome zu unterschiedlichen, im Rahmen der vorliegenden Erfindung liegenden Verbindungen der Formel I umgesetzt werden. Diese Halogenierungs- und Halogenaustauschreaktionen erfolgen gemäss an sich bekannten Arbeitsweisen. So kann man z.B. in der Gruppe R befindliche Wasserstoffatome mittels Halogenierungsreaktionen, z.B. Chlorierung oder Bromierung, durch Halogenatome substituierten. Die Halogenaustauschreaktionen in der Gruppe R erfolgen bevorzugt zwecks Einführung von Fluoratomen, beispielsweise durch Austausch von Halogenatomen, wie z.B. Chloratomen, durch Fluoratome unter Verwendung geeigneter Metallfluoride.

Beispiel 1: (Herstellung der Ausgangsverbindungen)

Herstellung von 2,2,4,4,4-Pentachlorbutyraldehyd:

a) In 200 ml Dimethylformamid werden unter Kühlung 10 g HCl-Gas eingeleitet und hierauf bei 60—65°C 10 g Chlor. Zu der schwach gelben Lösung wird ebenfalls bei 60—65°C die Lösung von 210 g 2,4,4,4-Tetrachlorbutyraldehyd in 300 ml Dimethylformamid zugetropft, und gleichzeitig wird eine ungefähr aliquote Menge Chlor eingeleitet, bis die Reaktionslösung schwach gelb gefärbt bleibt. Anschliessend wird die Temperatur noch 1 Stunde bei 65°C gehalten und dann das Reaktionsgemisch einer Wasserdampfdestillation unterworfen. Die organische Phase des Destillats wird abgetrennt und im Vakuum rektifiziert. Das bei $Kp._{15mm}$ 95—99°C siedene hellgelbe Oel wird aufgefangen.

b) 145,3 g Vinylidenchlorid, 148,0 g Trichloracetaldehyd, 3,0 g Kupfer(I)chlorid und 300 ml Acetonitril werden in einem Emaille-Autoklaven während 12 Stunden auf 125° erhitzt. Das Lösungsmittel wird hierauf am Wasserstrahlvakuum abdestilliert und der Rückstand in 500 ml Diäthyläther aufgenommen. Nach dem Abfiltrieren von ausgefallenem Kupferschlamm wird der Diäthyläther abdestilliert und der Rückstand am Wasserstrahlvakuum rektifiziert. Man erhält die Titelverbindung vom $Kp._{15mm}$ 95—99°C.

Herstellung von 2,2,4,4-Tetrachlorbutyraldehyd

125,0 g Vinylchlorid, 148,0 g Trichloracetaldehyd, 3 g Kupfer(I)-chlorid und 300 ml Acetonitril werden in einem Emaille-Autoklaven während 4 Stunden auf 140° erhitzt. Nach dem Erkalten, wird das Lösungsmittel am Wasserstrahlvakuum bei Raumtemperatur abdestilliert. Der Rückstand wird in 500 ml Diäthyläther aufgenommen und vom ausgefallenen Kupfer(I)chlorid abfiltriert. Nach dem Abdestillieren des Diäthyläthers wird der Rückstand am Wasserstrahlvakuum rektifiziert. Man erhält eine farblose Flüssigkeit vom $Kp._{12mm}$ 78—80°C.

Herstellung von 2,2,4-Trichlorbutyraldehyd:

56,1 g Aethylen, 148,0 g Trichloracetaldehyd, 3,0 g Kupfer(I)chlorid und 300 ml Acetonitril werden in einem Emaille-Autoklaven während 4 Stunden auf 140° erhitzt. Nach dem Erkalten wird das Lösungsmittel am Wasserstrahlvakuum bei Raumtemperatur abdestilliert. Der Rückstand wird in 500 ml Diäthyläther aufgenommen und vom ausgefallenen Kupfer(I)chlorid abfiltriert. Nach dem Abdestillieren des Diäthyläthers wird der Rückstand am Wasserstrahlvakuum rektifiziert. Man erhält eine farblose Flüssigkeit vom $Kp._{15mm}$ 64—66°C.

Beispiel 2: Herstellung von 2,3-Dichlor-5-(2,2,2-trichloräthyl)pyridin

244,3 g des gemäss Beispiel 1 hergestellten 2,2,4,4,4-Pentachlorbutyraldehyds werden mit 110 g Acrylnitril, 400 ml Acetonitril und 5 g CuCl in einem Tantal-Autoklaven während 4 Stunden auf 180°C erhitzt. Nach dem Erkalten werden das Acetonitril und der Ueberschuss an Acrylnitril im Vakuum abdestilliert. Das zurückbleibende dunkle Oel wird mit Diäthyläther extrahiert, der Aether mit $Na_2SO_4$ getrocknet und im Vakuum abgedampft. Der Rückstand wird einer Wasserdampfdestillation unterworfen. Das 2,3-Dichlor-5-(2,2,2-trichloräthyl)-pyridin fällt im Destillat in Form von fast weissen Blättchen aus. Nach einmaliger Umkristallisation aus mit Wasser verdünntem Aethanol hat das Produkt einen Smp. vom 98—99°C.

Beispiel 3: Herstellung von 2,3-dichlor-5-(2-chloräthyl)-pyridin

175,4 g 2,2,4-Trichlorbutyraldehyd werden mit 132,5 g Acrylnitril, 400 ml Acetonitril und 5 g CuCl in einem Tantal-Autoklaven innerhalb von 3 Stunden auf 180° erhitzt und 2 Stunden bei dieser Temperatur gehalten. Nach dem Erkalten wird das Acetonitril und der Ueberschuss an Acrylnitril im Vakuum abdestilliert. Das zurückbleibende dunkle Oel wird mit Diäthyläther extrahiert, der Aether mit $Na_2SO_4$ getrocknet und im Vakuum abgedampft. Der Rückstand wird einer Wasserdampfdestillation unterworfen.

Das 2,3-Dichlor-5-(2-chloraethyl)-pyridin fällt im Destillat in Form eines farblosen Oels an. Es wird im Hochvakuum destilliert und hat einem $Kp._{0,1mm}$ von 97—100°C.

Beispiel 4: Herstellung von 2,3-Dichlor-5-(pentachloräthyl)pyridin

a) 279,4 g 2,3-Dichlor-5-(2,2,2-trichloräthyl)-pyridin werden in 3 l Tetrachlorkohlenstoff gelöst. Unter Kühlung werden in diese Lösung innert ca.1 Stunde 120 g HCl-Gas eingeleitet, wobei teilweise das Hydrochlorid der Pyridinverbindung ausfällt. Das Reaktionsgemisch wird auf 50°C erwärmt und unter Belichtung mit einer Hg-Hochdrucklampe (125 Watt) mit Chlor-Gas behandelt.

Die Reaktionslösung wird hierauf im Vakuum eingedampft und der zurückbleibende Kristallbrei aus Methanol umkristallisiert. Es werden farblose Kristalle der Titelverbindung vom Smp. 97,5—98°C erhalten.

b) 210,5 g 2,3-Dichlor-5-(2-chloräthyl)-pyridin (erhalten gemäss Beispiel 3) werden nach der vorstehend unter a) angegebenen Methode chloriert und aufgearbeitet. Der Produkt hat einen Smp. von 97—98°C und ist mit dem vorstehend nach a) erhaltenen identisch.

c) 244,9 g 2,3-Dichlor-5-(2,2-dichloräthyl)-pyridin (erhalten gemäss dem nachstehenden Beispiel 7) werden nach der unter a) angegebenen Methode chloriert und aufgearbeitet. Das Produkt ist mit dem vorstehend nach a) erhaltenen identisch.

Beispiel 5: Herstellung von 2,3-Dichlor-5-(1,1,2,2-tetrafluor-2-chlor-äthyl)pyridin

348,2 g 2,3-Dichlor-5-(pentachloräthyl)-pyridin werden mit 1000 g Antimontrifluorid und 30 g Antimonpentachlorid während 2 Stunden bei 210°C verschmolzen. Nach dem Abkühlen auf 90°C wird die Schmelze mit 2 Liter Wasser versetzt und einer Wasserdampfdestillation unterworfen. Das überdestillierende farblose Oel wird abgetrennt, mit $Na_2SO_4$ getrocknet und im Wasserstrahlvakuum rektifiziert. Das beim $Kp._{11mm}$ 97—100°C destillierende Produkt wird aufgefangen.

Beispiel 6: Herstellung von 2,3-Dichlor-5-(1,1,2-trifluor-2,2-dichloräthyl)-pyridin

Der Destillationsrückstand gemäss dem vorangehenden Beispiel 5 wird weiterdestilliert und das beim $Kp._{11mm}$ 120—122°C übergehende Produkt aufgefangen.

Beispiel 7: Herstellung von 2,3-Dichlor-5-(2,2-dichloräthyl)-pyridin

Wird im vorstehenden Beispiel 3 der 2,2,4-Trichlorbutyraldehyd durch 210,0 g 2,2,4,4-Tetrachlorbutyraldehyd ersetzt und im übrigen analog verfahren, so erhält man das 2,3-Dichlor-5-(2,2-dichloraethyl)-pyridin in Form weisser Kristalle vom Smp. 89—90°C.

Analog den vorstehend beschriebenen Arbeitsweisen ist auch die folgende Verbindung der Formel I erhältlich:

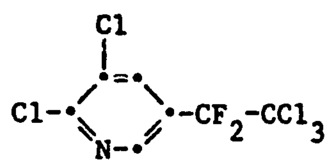

$Kp._{0,01mm}$ 72—73°C — wird beim Stehen kristallin.

**Patentansprüche**

1. Verbindung der Formel I

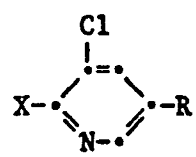

(I),

worin R einen einheitlich oder uneinheitlich mit 1—21 Halogenatomen substituierten $C_2$—$C_{10}$-Alkylrest und X Halogen bedeuten.

2. Verbindungen der Formel I gemäss Anspruch 1, dadurch gekennzeichnet, dass R einen einheitlich oder uneinheitlich mit 1—21 Fluor-, Chlor- oder Bromatomen substituierten $C_2$—$C_{10}$-Alkylrest und X Fluor, Chlor oder Brom bedeuten.

3. Verbindungen der Formel I gemäss Anspruch 2, dadurch gekennzeichnet, dass R einen einheitlich oder uneinheitlich mit 1—5 Fluor- oder Chloratomen substituierten Aethylrest und X Fluor oder Chlor bedeuten.

4. Verbindung der Formel I gemäss Anspruch 3, dadurch gekennzeichnet, dass R einen der Reste $—CH_2—CF_3$, $—CF_2—CF_2Cl$, $—CF_2—CFCl_2$, $—CCl_2—CCl_3$, $—CF_2—CCl_3$, $—CF_2—CH_3$, $—CCl_2—CH_3$, $—CF_2—CF_3$, $—CH_2—CH_2Cl$, $—CH_2—CHCl_2$ oder $—CH_2—CCl_3$ bedeutet.

5. Verbindung der Formel I gemäss Anspruch 1 bis 4, dadurch gekennzeichnet, dass X Chlor bedeutet.

6. Verbindung gemäss Anspruch 4 der Formel

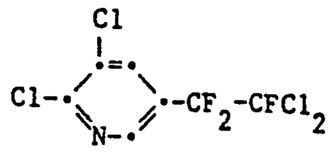 .

7. Verbindung gemäss Anspruch 4 der Formel

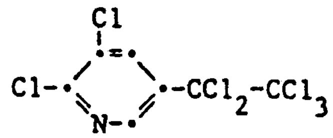 .

8. Verbindung gemäss Anspruch 4 der Formel

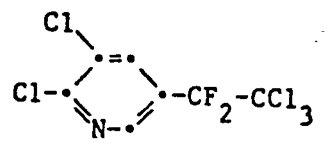 .

9. Verbindung gemäss Anspruch 4 der Formel

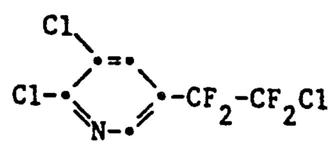 .

10. Verbindung gemäss Anspruch 4 der Formel

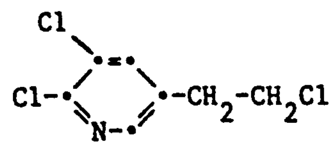 .

11. Verbindung gemäss Anspruch 4 der Formel

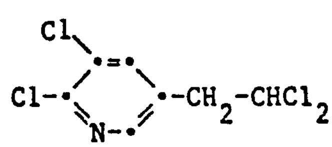 .

12. Verbindung gemäss Anspruch 4 der Formel

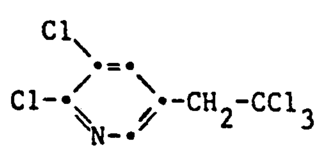 .

13. Verfahren zur Herstellung einer Verbindung der Formel I gemäss Anspruch 1 bis 12, dadurch gekennzeichnet, dass man eine Verbindung der Formel II

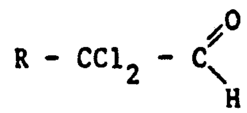 (II)

mit Acrylnitril mittels einer Anlagerungsreaktion zu einem Zwischenprodukt der Formel III

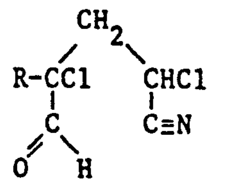 (III)

umsetzt und das Zwischenprodukt der Formel III in Gegenwart eines Halogenwasserstoffs HX cyclisiert, wobei R und X die in den Ansprüchen 1 bis 5 angegebenen Bedeutungen haben, und dass man gegebenenfalls die erhaltene Verbindung der Formel I im Rest R halogeniert oder im Rest R befindliche Halogenatome durch andere Halogenatome austauscht.

14. Verfahren nach Anspruch 13 zur Herstellung einer Verbindung der Formel I, worin X Chlor bedeutet, dadurch gekennzeichnet, dass man eine direkte Umsetzung ohne Isolierung des Zwischenproduktes der Formel III in situ vornimmt und dass man ohne Zusatz eines Halogenwasserstoffs HX arbeitet oder als Halogenwasserstoff HCl verwendet.

15. Verfahren gemäss Anspruch 13 oder 14, dadurch gekennzeichnet, dass man die erhaltene Verbindung der Formel I im Rest R chloriert oder fluoriert.

16. Verfahren nach Anspruch 13, dadurch gekennzeichnet, dass man die Anlagerungsreaktionen bei einer Temperatur von 70—160°C durchführt.

17. Verfahren nach Anspruch 13, dadurch gekennzeichnet, dass man die Anlagerungsreaktionen in einem geschlossenen System bei einer Temperatur von 70—160°C und einem der angewendeten Reaktionstemperatur entsprechenden Druck durchführt.

18. Verfahren nach Anspruch 13, dadurch gekennzeichnet, dass man die Cyclisierung der Verbindungen der Formel III bei einer Temperatur zwischen 0 und 220°C, vorzugsweise 100 und 200°C, durchführt.

19. Verfahren nach Anspruch 13, dadurch gekennzeichnet, dass man die Cyclisierung der Verbindungen der Formel III bei einer Temperatur zwischen 100 und 200°C in einem offenen System in Gegenwart von Chlorwasserstoff oder in Gegenwart von Substanzen, welche unter den Reaktionsbedingungen Chlorwasserstoff bilden, durchführt.

20. Verfahren nach Anspruch 13, dadurch gekennzeichnet, dass man die Cyclisierung der Verbindungen der Formel III in Abwesenheit eines Lösungsmittels durch Erhitzen in der Flüssig- oder in der Gasphase vornimmt.

21. Verfahren nach Anspruch 13, dadurch gekennzeichnet, dass man die Verbindungen der Formel III zunächst isoliert und anschliessend in einer zweiten Verfahrensstufe cyclisiert.

22. Verfahren nach Anspruch 13 oder 14, dadurch gekennzeichnet, dass man die Umsetzung in Gegenwart eines Katalysators durchführt.

23. Verfahren nach Anspruch 22, dadurch gekennzeichnet, dass man die Umsetzung bei einer Temperatur zwischen 70 und 220°C, insbesondere zwischen 130 und 200°C, durchführt.

24. Verfahren nach Anspruch 22, dadurch gekennzeichnet, dass man die Umsetzung in einem geschlossenen System bei einem der jeweiligen Reaktionstemperatur entsprechenden Druck durchführt.

25. Verfahren nach Anspruch 22, dadurch gekennzeichnet, dass man als Katalysator Kupferpulver, Kupferbronze, Kupfer(I)- oder Kupfer(II)chlorid bzw. -bromid, Kupfer(I)jodid oder deren Gemische verwendet.

26. Verfahren nach Anspruch 22, dadurch gekennzeichnet, dass man den Katalysator in Mengen von 0,01 bis 10 Mol%, vorzugsweise 0,1 bis 6 Mol%, bezogen auf den Aldehyd, verwendet.

27. Verfahren nach Anspruch 13 oder 14, dadurch gekennzeichnet, dass man die Umsetzung in Gegenwart eines inerten organischen Lösungsmittels durchführt.

28. Verfahren nach Anspruch 27, dadurch gekennzeichnet, dass man die Anlagerungsreaktionen bei einer Temperatur von 70—160°C in Acetonitril, Butyronitril oder 3-Methoxypropionitril als Lösungsmittel in Gegenwart von 0,1—6 Mol% Kupferpulver, Kupferbronze, Kupfer(I)- oder Kupfer(II)chlorid bzw. -bromid oder Kupfer(I)jodid oder in Gegenwart eines Gemisches dieser Substanzen in einem geschlossenen System durchführt und die erhaltene Verbindung der Formel III bei einer Temperatur zwischen 100 und 200°C in einem offenen System in Gegenwart von Halogenwasserstoff oder einer Substanz, welche unter den Reaktionsbedingungen Halogenwasserstoff bildet, zu einer Verbindung der Formel I cyclisiert.

29. Verfahren nach Anspruch 27, dadurch gekennzeichnet, dass man die Umsetzung in einem Alkancarbonsäurenitril mit 2—5 Kohlenstoffatomen, einem 3-Alkoxypropionitril mit 1—2 Kohlenstoffatomen in der Alkoxygruppe oder in überschüssigem Acrylnitril als Lösungsmittel durchführt.

30. Verfahren nach Anspruch 22, dadurch gekennzeichnet, dass man die Umsetzung in Acetonitril, Butyronitril oder 3-Methoxypropionitril als Lösungsmittel in Gegenwart von 0,1—6 Mol% Kupferpulver, Kupferbronze, Kupfer(I)- oder Kupfer(II)-chlorid bzw. -bromid oder Kupfer(I)jodid oder einem Gemisch dieser Substanzen bei 130 bis 200°C in einem geschlossenen System bei einem der jeweils angewendeten Reaktionstemperatur entsprechenden Druck vornimmt.

31. Verbindung der Formel II

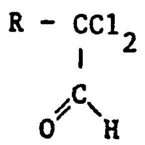

(II),

worin R einen der Reste $—CH_2—CCl_3$, $—CH_2—CHCl_2$ oder $—CH_2—CH_2Cl$ bedeutet.

32. Verfahren zur Herstellung einer Verbindung der Formel II gemäss Anspruch 31, dadurch gekennzeichnet, dass man Trichloracetaldehyd mit einer Verbindung der Formel IV

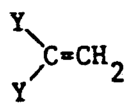

(IV)

umsetzt, wobei Y Wasserstoff oder Chlor bedeutet.

**Revendications**

1. Composé de formule I

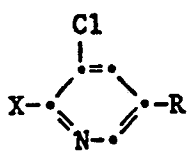

(I),

où R représente un radical alkyle en $C_{2-10}$ substitué de manière homogène ou non-homogène avec de 1 à 21 atomes de carbone et X représente un halogène.

2. Composés de formule I, selon la revendication A, caractérisés en ce que R représente un radical alkyle en $C_{2-10}$ substitué de manière homogène ou non-homogène avec de 1 à 21 atomes de fluor, de chlore ou de brome et X représente un fluor, un chlore ou un brome.

3. Composés de formule I selon la revendication 2, caractérisés en ce que R représente un radical éthyle substitué de façon homogène ou non-homogène avec de 1 à 5 atomes de fluor ou de chlore et X représente un fluor ou un chlore.

4. Composé de formule I selon la revendication 3, caractérisé en ce que R représente l'un des radicaux $—CH_2—CF_3$, $—CF_2—CF_2Cl$, $—CF_2—CFCl_2$, $—CCl_2—CCl_3$, $—CF_2—CCl_3$, $—CF_2—CH_3$, $—CCl_2—CH_3$, $—CF_2—CF_3$, $—CH_2—CH_2Cl$, $—CH_2—CHCl_2$ ou $—CH_2—CCl_3$.

5. Composé de formule I selon l'une des revendications 1 à 4, caractérisé en ce que X représente un chlore.

6. Composé selon la revendication 4, de formule

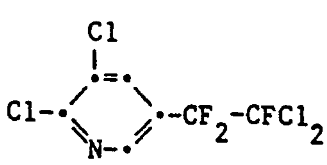

.

7. Composé selon la revendication 4, de formule

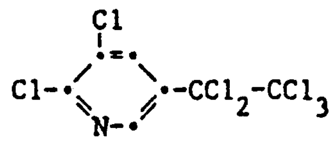 .

8. Composé selon la revendication 4, de formule

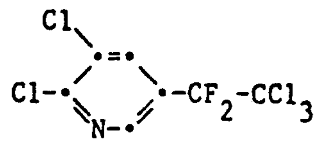 .

9. Composé selon la revendication 4, de formule

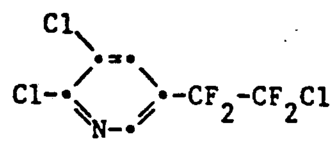 .

10. Composé selon la revendication 4, de formule

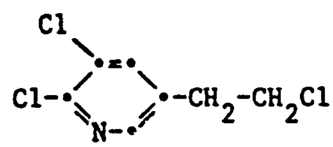 .

11. Composé selon la revendication 4, de formule

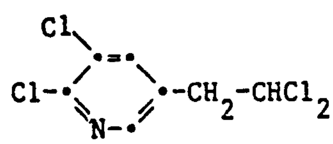 .

12. Composé selon la revendication 4, de formule

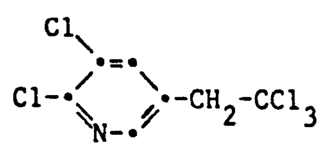 .

13. Procédé de préparation d'un composé de formule I, selon les revendications 1 à 12, caractérisé en ce qu'on fait réagir un composé de formule II

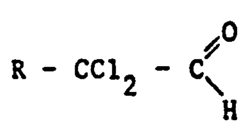 (II)

avec de l'acrylonitrile au moyen d'une réaction d'addition pour donner un produit intermédiaire de formule III

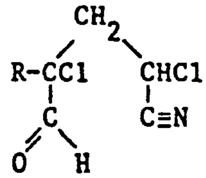

(III)

et en ce qu'on cyclise le produit intermédiaire de formule III en présence d'un acide halohydrique HX, où R et X ont les significations données dans les revendications 1 à 5, et en ce que éventuellement on halogène le composé de formule I obtenu dans le radical R ou bien en ce qu'on échange les atomes d'halogène se trouvant dans le radical R contre des atomes d'halogène.

14. Procédé selon la revendication 13, de préparation d'un composé de formule I où X représente un chlore, caractérisé en ce qu'on procède à une réaction direct *in situ* sans isoler le produit intermédiaire de formule III et en ce qu'on travaille sans ajouter un acide halohydrique HX ou en ce qu'on utilise comme acide halohydrique HCl.

15. Procédé selon la revendication 13 ou 14, caractérisé en ce qu'on chlore ou un ce qu'on fluore le composé de formule I obtenu dans le radical R.

16. Procédé selon la revendication 13, caractérisé en ce qu'on conduit la réaction d'addition à une température de 70—160°C.

17. Procédé selon la revendication 13, caractérisé en ce qu'on conduit la réaction d'addition dans un système fermé à une température de 70—160°C et une pression correspondante à la température réactionnelle utilisée.

18. Procédé selon la revendication 13, caractérisé en ce qu'on procède à la cyclisation de composés de formule III à une température comprise entre 0 et 120°C, de préférence 100 et 200°C.

19. Procédé selon la revendication 13, caractérisé en ce qu'on procède à la cyclisation des composés de formule III à une température comprise entre 100 et 200°C dans un système ouvert en présence d'acide chlorhydrique ou en présence de substances qui forment de l'acide chlorhydrique dans les conditions de la réaction.

20. Procédé selon la revendication 13, caractérisé en ce qu'on conduit la cyclisation des composés de formule III en présence d'un solvant par chauffage en phase liquide ou en phase gazeuse.

21. Procédé selon la revendication 13, caractérisé en ce qu'on isole tout d'abord les composés de formule III et en ce qu'on cyclise dans une seconde étape.

22. Procédé selon la revendication 13 ou 14, caractérisé en ce qu'on conduit la réaction en présence d'un catalyseur.

23. Procédé selon la revendication 22, caractérisé en ce qu'on conduit la réaction à une température comprise entre 70 et 220°C, en particulier entre 130 et 200°C.

24. Procédé selon la revendication 22, caractérisé en ce qu'on conduit la réaction dans un système fermé à une pression correspondante à la température réactionnelle considérée.

25. Procédé selon la revendication 22, caractérisé en ce qu'on utilise comme catalyseur la poudre de cuivre, un mélange pulvérulent de cuivre et de bronze, le chlorure ou le bromure de cuivre (I) ou de cuivre (II), l'iodure de cuivre (I) ou leurs mélanges.

26. Procédé selon la revendication 22, caractérisé en ce qu'on utilise le catalyseur à des quantités de 0,01 à 10% molaires, de préférence 0,1 à 6% molaires, par rapport à l'aldéhyde.

27. Procédé selon la revendication 13 ou 14, caractérisé en ce qu'on conduit la réaction en présence d'un solvant organique inerte.

28. Procédé selon la revendication 27, caractérisé en ce qu'on conduit la réaction d'addition à une température de 70—160°C dans l'acétonitrile, le butyronitrile ou le 3-méthoxypropionitrile comme solvant en présence de 0,1—6% molaires de poudre de cuivre, de mélange pulvérulent de cuivre et de bronze, de chlorure ou de bromure de cuivre (I) ou de cuivre (II) ou d'iodure de cuivre (I) ou en présence d'un mélange de ces substances dans un système fermé et en ce qu'on cyclise le composé de formule (III) obtenu à une température comprise entre 100 et 200°C dans un système ouvert en présence d'un acide halohydrique ou d'une substance qui forme un acide halohydrique dans les conditions de la réaction, pour donner un composé de formule (I).

29. Procédé selon la revendication 27, caractérisé en ce qu'on conduit la réaction dans un nitrile d'acide alcanecarboxylique en $C_{2-5}$, dans un 3-alcoxypropionitrile en $C_{1-2}$ dans le groupe alcoxy ou dans un excès d'acrylonitrile comme solvant.

30. Procédé selon la revendication 22, caractérisé en ce qu'on conduit la réaction dans l'acétonitrile, le butyronitrile ou le 3-méthoxypropionitrile comme solvant en présence de 0,1—6% molaires de poudre de cuivre, de mélange pulvérulent de cuivre et de bronze, de chlorure ou de bromure de cuivre (I) ou de cuivre (II) ou d'iodure de cuivre (I) ou un mélange de ces substances entre 130 et 200°C dans un système fermé à

une pression correspondant à la température réactionnelle utilisée.

31. Composé de formule II

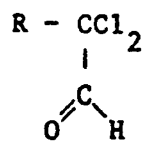

(II),

où R représente l'un des radicaux $—CH_2—CCl_3$, $—CH_2—CHCl_2$ ou $—CH_2—CH_2Cl$.

32. Procédé de préparation d'un composé de formule II selon la revendication 31, caractérisé en ce qu'on fait réagir le trichloracétaldéhyde avec un composé de formule IV

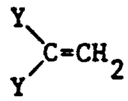

(IV)

où Y représente un hydrogène ou un chlore.

**Claims**

1. A compound of formula I

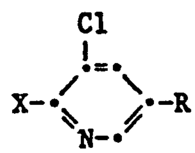

(I)

wherein R is a $C_2—C_{10}$ alkyl group which is uniformly or non-uniformly substituted by 1 to 21 halogen atoms, and X is halogen.

2. Compounds of formula I according to claim 1, wherein R is a $C_2—C_{10}$ alkyl group which is uniformly or non-uniformly substituted by 1 to 21 fluorine, chlorine or bromine atoms, and X is fluorine, chlorine or bromine.

3. Compounds of formula I according to claim 2, wherein R is an ethyl group which is uniformly or non-uniformly substituted by 1 to 5 fluorine or chlorine atoms, and X is fluorine or chlorine.

4. A compound of formula I according to claim 3, wherein R is one of the radicals $—CH_2—CF_3$, $—CF_2—CF_2Cl$, $—CF_2—CFCl_2$, $—CCl_2—CCl_3$, $—CF_2—CCl_3$, $—CF_2—CH_3$, $—CCl_2—CH_3$, $—CF_2—CF_3$, $—CH_2—CH_2Cl$, $—CH_2—CHCl_2$ or $—CH_2—CCl_3$.

5. A compound of formula I according to claims 1 to 4, wherein X is chlorine.

6. A compound according to claim 4 of the formula

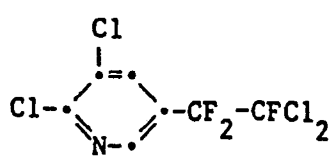

.

7. A compound according to claim 4 of the formula

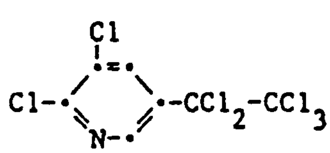

,

8. A compound according to claim 4 of the formula

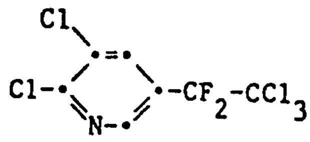 .

9. A compound according to claim 4 of the formula

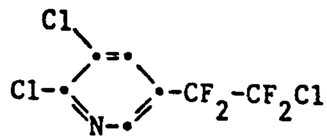 .

10. A compound according to claim 4 of the formula

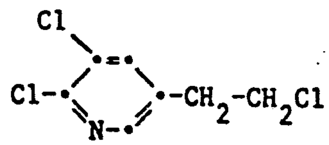 .

11. A compound according to claim 4 of the formula

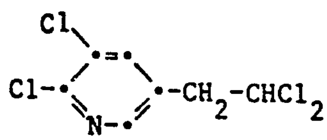 .

12. A compound according to claim 4 of the formula

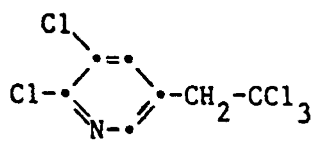 .

13. A process for producing a compound of formula I according to claims 1 to 12, which process comprises reacting a compound of formula II

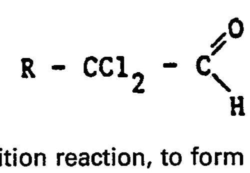 (II)

with acrylonitrile, by means of an addition reaction, to form an intermediate of formula III

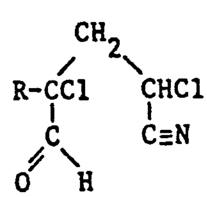 (III)

and cyclising the intermediate of formula III in the presence of a hydrogen halide HX, wherein R and X have the meanings given in claims 1 to 5, and optionally halogenating the resulting compound of formula I in the radical R, or exchanging halogen atoms present in the radical R for other halogen atoms.

14. A process according to claim 13 for producing a compound of formula I wherein X is chlorine, which process comprises carrying out a direct reaction *in situ*, without isolation of the intermediate of

formula III, and performing the reaction without the addition of a hydrogen halide HX, or using HCl as hydrogen halide.

15. A process according to claim 13 or 14, wherein the resulting compound of formula I is chlorinated or fluorinated in the radical R.

16. A process according to claim 13, wherein the addition reactions are performed at a temperature of 70—160°C.

17. A process according to claim 13, wherein the addition reactions are performed in a closed system at a temperature of 70—160°C, and under a pressure corresponding to the reaction temperature applied.

18. A process according to claim 13, wherein the cyclisation of the compounds of formula III is performed at a temperature of between 0 and 220°C, preferably between 100 and 200°C.

19. A process according to claim 13, wherein the cyclisation of the compounds of formula III is performed at a temperature of between 100 and 200°C in an open system, in the presence of hydrogen chloride, or in the presence of substances which form hydrogen chloride under the reaction conditions.

20. A process according to claim 13, wherein the cyclisation of the compounds of formula III is performed, in the absence of a solvent, by heating in the liquid phase or in the gas phase.

21. A process according to claim 13, wherein the compounds of formula III are first isolated, and subsequently cyclised in a second stage of the process.

22. A process according to claim 13 or 14, wherein the reaction is performed in the presence of a catalyst.

23. A process according to claim 22, wherein the reaction is performed at a temperature of between 70 and 220°C, especially between 130 and 200°C.

24. A process according to claim 22, wherein the reaction is performed in a closed system under a pressure corresponding to the particular reaction temperature applied.

25. A process according to claim 22, wherein the catalyst used is copper powder, copper bronze, copper(I) or copper(II) chloride or bromide, copper(I) iodide, or a mixture thereof.

26. A process according to claim 22, wherein the catalyst is used in an amount of 0.01 to 10 mol %, preferably 0.1 to 6 mol %, relative to the aldehyde.

27. A process according to claim 13 or 14, wherein the reaction is performed in the presence of an inert organic solvent.

28. A process according to claim 27, wherein the addition reactions are performed at a temperature of 70—160°C in acetonitrile, butyronitrile or 3-methoxypropionitrile as solvent, in the presence of 0.1 to 6 mol % of copper powder, copper bronze, copper(I) or copper(II) chloride or bromide or copper(I) iodide, or in the presence of a mixture of these substances with one another, and in a closed system, and the resulting compound of formula III is cyclised to a compound of formula I at a temperature of between 100 and 200°C in an open system and in the presence of a hydrogen halide or of a substance which forms a hydrogen halide under the reaction conditions.

29. A process according to claim 27, wherein the reaction is performed in an alkanecarboxylic acid nitrile having 2—5 carbon atoms, in a 3-alkoxypropionitrile having 1—2 carbon atoms in the alkoxy group, or in excess acrylonitrile, as the solvent.

30. A process according to claim 22, wherein the reaction is performed in acetonitrile, butyronitrile or 3-methoxypropionitrile, as the solvent, in the presence of 0.1—6 mol % of copper powder, copper bronze, copper(I) or copper(II) chloride or bromide or copper(I) iodide, or in a mixture of these substances with one another, at 130 to 200°C, and in a closed system under a pressure corresponding to the particular reaction temperature applied.

31. A compound of the formula II

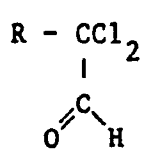

(II)

wherein R is any one of the radicals $—CH_2—CCl_3$, $—CH_2—CHCl_2$ and $—CH_2—CH_2Cl$.

32. A process for producing a compound of formula II according to claim 31, which comprises reacting trichloroacetaldehyde with a compound of formula IV

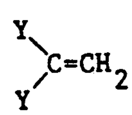

(IV)

wherein Y is hydrogen or chlorine.